Europäisches Patentamt

**European Patent Office**

**Office européen des brevets**

(11) Publication number: **0 155 747**

**A1**

## EUROPEAN PATENT APPLICATION

(21) Application number: 85300163.4

(22) Date of filing: 10.01.85

(51) Int. Cl.⁴: **C 12 M 1/30**

(30) Priority: 19.03.84 US 591336

(43) Date of publication of application:
25.09.85 Bulletin 85/39

(84) Designated Contracting States:
DE FR GB IT

(71) Applicant: KIDDE, INC.
Park 80 West Plaza Two Box 5555
Saddle Brook, NJ 07662(US)

(72) Inventor: Walter, Carl W.
91, Highland Street
Holliston Massachusetts, 01746(US)

(72) Inventor: Woodworth, John L.
2, Whalen Road
Hopkinton Massachusetts, 01748(US)

(74) Representative: Kerr, Simonne June et al,
European Patent Attorney POTTS, KERR & CO. 13 High
Street
Windsor Berkshire SL4 1LD(GB)

(54) Tube culture system.

(57) A combined swab and culture storage receptacle (11) including a first container (12) comprising a rupturable wall portion (16) and defining a first sealed chamber (13), a culture-sustaining media (31) retained in the first chamber, a second container (14) defining a second chamber (15) separated from the first chamber (13) by the rupturable wall portion (16) and a closure (18) providing access to the second chamber. Retained in the second chamber (15) is a swab (23) comprising a shaft portion (25) and a head portion (24) disposed directly adjacent to the rupturable wall portion (16). The swab (23) is movable within the second chamber (15) so as to permit the head portion (24) to rupture the rupturable wall portion (16) and enter the first chamber (13). After employment of the swab (23) to collect a culture specimen, the head portion (24) is forced through the rupturable wall portion (16) into the first chamber (13) for immersion in the culture-sustaining media (31).

*FIG.2*

## SEALED TUBE CULTURE SYSTEM

This invention relates generally to a two compartment receptacle and, more specifically, to such a receptacle for the collection and storage of medical culture specimens.

Medical diagnostics frequently entail the collection from patients of culture specimens. Customarily, such specimens are obtained by swabbing a designated body area with an absorbent swab. The specimen then is transported to a laboratory for analysis. However, to ensure a correct analysis, the collected specimen must be preserved in substantially its original condition. For that reason, a culture retaining swab usually is kept in a suitable preservative solution during that period between the collection of the culture and its subsequent analysis. Such preservative solutions commonly are known as culture-sustaining media.

The prior art includes various receptacles for storing culture retaining swabs in a culture-sustaining media. In many cases, the swab comprises a cap that closes and seals an open end of the receptacle upon insertion thereinto. The swab then remains sealed in the media filled receptacle until it is removed for analysis. One well known swab receptacle consists of a flexible plastic tube containing a media filled glass vile. Prior to insertion of the swab, the plastic tube is squeezed to break the vile and release the media.

Prior culture retaining swab receptacles have suffered from a number of disadvantages. For example, some render difficult the maintenance of sterile conditions for a collected culture specimen. Others fail under certain circumstances to maintain a culture specimen immersed in culture-sustaining media and thereby detrimentally affect the specimen prior to analysis. Those employing frangible media-filled viles have the obvious disadvantages inherent in the glass fragments produced during their use.

The object of this invention, therefore, is to provide an improved receptacle for swabs and the culture specimens collected there-with.

The invention is a combined swab and culture storage receptacle including a first container comprising a rupturable wall portion and defining a first sealed chamber, a culture-sustaining media retained in the first chamber, a second container defining a second chamber separated from the first chamber by the rupturable wall portion and a closure providing access to the second chamber. Retained in the second chamber is a swab comprising a shaft portion and a head portion disposed directly adjacent to the rupturable wall portion. The swab is movable within the second chamber so as to permit the head portion to rupture the rupturable wall portion and enter the first chamber. After employment of the swab to collect a culture specimen, the head portion is forced through the rupturable wall portion into the first chamber for immersion in the culture-sustaining media. After entry into the first chamber, the swab head portion remains immersed in the culture-sustaining media until a laboratory analysis of the specimen can be made

The second container and the rupturable wall portion may be integrally molded with the first container which comprises flexible wall portions and is sealed after being filled with the culture-sustaining media. This arrangement simplifies construction and thereby provides an efficient receptacle at relatively low cost.

The second container may consist of an elongated housing having one end closed by the rupturable wall portion and an opposite end closed by the closure which retains an end of the swab shaft portion opposite to an end retaining the head portion. This arrangement facilitates rupture of the rupturable wall portion and sealing of the second chamber after entry of a culture retaining swab.

The closure may comprise a cap threadedly engaged with the elongated housing and arranged such that a given degree of axial engagement therebetween produces movement of the swab head portion through the rupturable wall portion into the first chamber. The threaded cap and housing establish a mechanically advantaged mechanism for forcing the swab head portion through the rupturable wall portion.

A stop may be provided for establishing between the cap and the housing a predetermined relative axial position that positions the head portion directly adjacent to the rupturable wall portion. The stop prevents inadvertent premature rupture of the rupturable wall portion but after collection of a culture can be circumvented by the application of sufficient torque between the cap and the elongated housing.

The swab head portion may comprise abrasive surface portions for collecting a culture to be tested. The use of a swab head portion with abrasive surface portions facilitates the collection of cultures that are not readily attainable with conventional absorbent swabs.

The invention will now be described further, by way of example, with reference to the accompanying drawings, in which:-

Fig. 1 is a schematic elevational view of a receptacle according to the invention;

Fig. 2 is a schematic cross sectional view taken along lines 2-2 of Fig. 1;

Fig. 3 is a schematic cross sectional view similar to that shown in Fig. 2 but after rupture of a dividing wall portion; and

Fig. 4 is a detailed view of a swab head portion shown in Figs. 2 and 3.

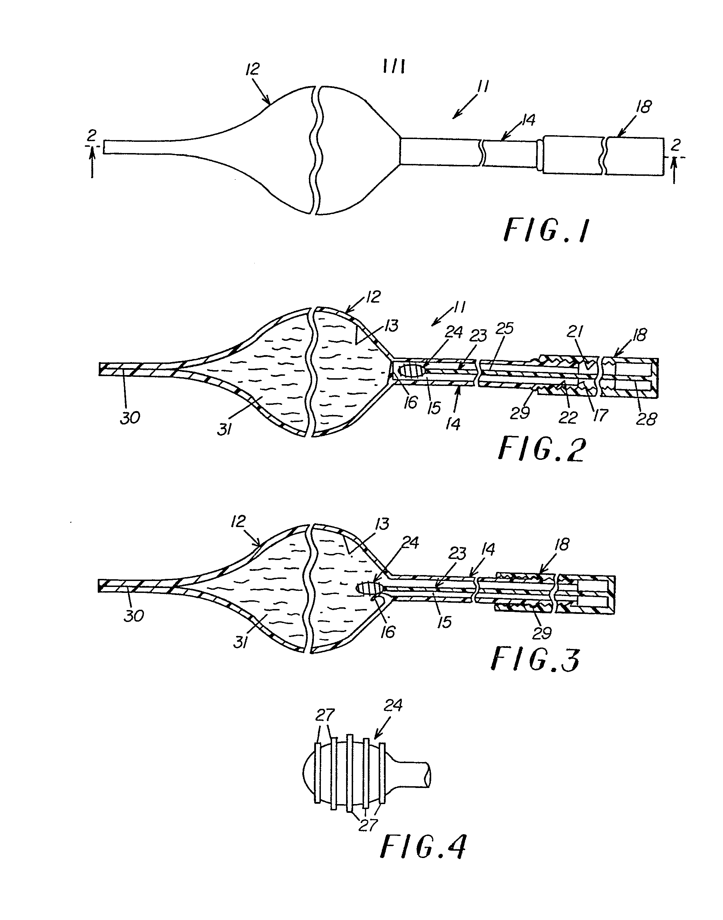

Illustrated in Figs. 1-3 is a combined swab and culture storage receptacle 11 according to the invention. The receptacle 11 includes a first container 12 defining a first chamber 13 and a second container 14 defining a second chamber 15. Separating the first chamber 13 from the second chamber 15 is a rupturable membrane wall portion 16 of the first container 12. As shown in Figs. 2 and 3, the second container 14 is an elongated housing having one end closed by the rupturable membrane 16 and an open opposite end 17 covered by a cap closure 18. The cap 18 has inner threads 21 that engage outer threads 22 on the housing 14.

Retained within the second chamber 15 is a swab 23 having a head portion 24 and a shaft portion 25. The head portion 24 is retained by one end of the shaft portion 25 and is disposed directly adjacent to the rupturable membrane 16. As shown most clearly in Fig. 4, the head portion possesses a plurality of spaced apart annular ridges 27. Preferably, the head portion 24 and the annular ridges 27 are integrally formed of a rigid plastic so as to provide abrasive surface portions along the edges of the ridges 27. The end 28 of the swab shaft portion 25 is retained by and preferably molded integrally with the threaded cap 18. Also formed on the outer surface of the container housing 14 directly adjacent to the inner termination of the outer threads 22 is an annular ridge stop.

During construction of the receptacle 11, the first container 12, the rupturable membrane 16 and the second container 14 preferably are integrally molded in plastic as the composite unit with the first container 12 initially having an open end 30. Subsequently, the first chamber 13 is filled with a suitable culture-sustaining nutrient solution 31. The open end 30 then is hermetically sealed by, for example, the application of heat and pressure. After insertion of the head portion 24 of the swab 23 into the second chamber 15, the cap 18 is screwed onto the housing 14. When the inner end of the cap 18 reaches the annular stop 19 the relative axial positions of the engaged housing 14 and cap 18 are such that the swab head portion 24 is closely adjacent to the rupturable membrane 16. This discernible engagement between the cap 18 and the ridge stop 9 apprises an assembler of that desired position and thereby prevents inadvertent premature rupture of the membrane 16.

The receptacle unit 11 is employed first to collect a culture specimen from a selected area of a body. At the time of use, the cap 18 is screwed off of the elongated housing 14 and the swab 23 is removed from the chamber 15. The abrasive edges of the ridges 27 then are used to scrape the desired culture specimen from the selected body area. After collection of the specimen, the swab is returned to the chamber 15 and the cap 18 screwed back onto the housing 14 until engagement of the ridge stop 9 is felt.

The application of sufficient torque between the cap 18 and the housing 14 then forces the inner threads 21 over the ridge stop 29. That action forces the swab head portion through the rupturable membrane 16 and into the chamber 13 for immersion in the culture-sustaining media 31. The engagements between both the inner and outer threads 21, 22 and the cap 18 and ridge stop 29 provide seals that isolate the sterile chamber 15 from the environment. Once the collected culture specimen has been sealed within the chamber 13, the receptacle 11 can be transported to a test laboratory.

Upon reaching a suitable test facility, the cap 18 again is disengaged by being screwed off of the housing 14. This permits the swab head portion 24 to be withdrawn easily from first the chamber 13 and then the chamber 15. Conventional test procedures then can be employed to analyze the culture specimen retained by the swab head portion 24 which specimen has been preserved by the media 31.

CLAIMS

1. A combined swab and culture collection receptacle (11) comprising a first container (12) comprising a rupturable wall portion (16) and defining a first sealed chamber (13), a culture-sustaining media (31) retained in the first sealed chamber (13), a second container (14) defining a second chamber (15) separated from the first chamber (13) by the rupturable wall portion (16) characterized in that a swab (23) is retained in the second chamber (15) and comprises a shaft portion (25) and a head portion (24) disposed directly adjacent to the rupturable wall portion (16), the swab (23) being movable within the second chamber (15) so as to permit the head portion (24) to rupture the rupturable wall portion (16) and enter the first chamber (13), a closure (18) providing access to the second chamber (15).

2. A receptacle according to claim 1, characterized in that the first chamber (13) comprises flexible wall portions.

3. A receptacle according to claim 2, characterized in that the rupturable wall portion (16) is an integrally molded portion of the first container (12).

4. A receptacle according to claim 3, characterized in that the first and second containers (12, 14) comprise an integrally molded plastic unit.

5. A receptacle according to claim 3, characterized in that the second container (14) comprises an elongated housing having one end closed by the rupturable wall portion (16) and an opposite end closed by the closure (18).

6. A receptacle according to claim 5, characterized in that the shaft portion (25) comprises one end retaining the head portion (24) and an opposite end retained by the closure (18).

7. A receptacle according to claim 6, characterized in that the closure (18) comprises a cap threadedly engaging the opposite end of the elongated housing (14).

8. A receptacle according to claim 7, characterized in that a given degree of axial engagement between the cap (18) and the housing (14) produces movement of the head portion (24) through the rupturable wall portion (16) into the first chamber (13).

9. A receptacle according to claim 8, characterized in that a stop (29) for establishing between the cap (18) and the housing (14) a predetermined relative axial position wherein the head portion (24), is disposed in the second chamber (15) directly adjacent to the rupturable wall portion (16).

10. A receptacle according to claim 9, characterized in that the stop comprises an annular ridge on the outer surface of the housing (14).

11. A receptacle according to claim 10, characterized in that the head portion (24) comprises abrasive surface portions (27) for collecting a culture to be tested.

0155747

FIG.1

FIG.2

FIG.3

FIG.4

## European Patent Office

**EUROPEAN SEARCH REPORT**

## DOCUMENTS CONSIDERED TO BE RELEVANT

| Category | Citation of document with indication. where appropriate, of relevant passages | Relevant to claim | CLASSIFICATION OF THE APPLICATION (Int Cl 4) |
|---|---|---|---|
| X | US-A-4 150 950  (M.T. TAKEGUCHI) <br> * Figures; column 2, line 54 - column 3, line 19 * | 1,4-8 | C 12 M   1/30 |
| | --- | | |
| X | US-A-3 923 604  (F. MONAGHAN) <br> * Column 2, lines 2-64; claims; figures * | 1,3-6 | |
| | --- | | |
| X | US-A-3 776 220  (F. MONAGHAN) <br> * Figures; column 2, line 2 - column 3, line 9 * | 1,3-6 | |
| | --- | | |
| X | GB-A-2 059 992  (BRIAN RANDALL PIKE) <br> * Figures * | 1-4 | |
| | --- | | **TECHNICAL FIELDS SEARCHED (Int Cl 4)** |
| A | GB-A-2 028 868  (PRECISION DYNAMICS CORP.) <br> * Figures; page 3, lines 42-52 * | 1,9 | C 12 M |
| | ----- | | |

The present search report has been drawn up for all claims

| Place of search | Date of completion of the search | Examiner |
|---|---|---|
| THE HAGUE | 06-05-1985 | COUCKE A.O.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82